# EUROPÄISCHE PATENTSCHRIFT

(11) **EP 1 773 204 B1**
(45) Veröffentlichungstag und Bekanntmachung des Hinweises auf die Patenterteilung: **02.01.2008**
(21) Anmeldenummer: 05761629.4
(22) Anmeldetag: 23.07.2005
(51) Int. Cl.: A61B 17/00

(54) **ENDOCHIRURGISCHER BERGEBEUTEL FÜR DIE AUFNAHME VON KÖRPERGEWEBE ODER -FLÜSSIGKEIT**
ENDOSURGICAL EXTRACTION BAG FOR RECEIVING BODILY TISSUE OR FLUID
SAC EXTRACTEUR POUR ENDOCHIRURGIE DESTINE A RECEVOIR UN TISSU OU UN LIQUIDE CORPOREL

(30) Priorität: 28.07.2004 DE 102004038071
(43) Veröffentlichungstag der Anmeldung: 18.04.2007
(73) Patentinhaber: MTP Medical Technical Promotion GmbH, 78579 Neuhausen ob Eck (DE)
(72) Erfinder: LEROY, Joel, F-62160 Bully les Mines (FR); MARESCAUX, Jacques, F-67310 Scharrachbergheim (FR); MUTTER, Didier, F-67550 Vendenheim (FR); VIX, Michel, F-67207 Niederhausbergen (FR)
(74) Vertreter: Heuckeroth, Volker
(86) Internationale Anmeldenummer: PCT/EP2005/008052
(87) Internationale Veröffentlichungsnummer: WO 2006/013031

(56) Entgegenhaltungen:
- EP-A- 0 578 997
- EP-A- 1 132 051
- US-B1- 6 206 889

## Beschreibung

Die Erfindung betrifft einen endochirurgischen Bergebeutel für die Aufnahme von Körpergewebe oder -flüssigkeit, mit einem Beutelhauptteil, der aus einer flexiblen Hülle gebildet ist, und mit einer ersten Einfüllöffnung zum Einbringen von Gewebe oder Flüssigkeit in den Beutelhauptteil.

Ein Bergebeutel der eingangs genannten Art ist aus dem Dokument EP 0 578 997 B1 bekannt.

Ein solcher Hergebeutel, der auch als Extraktionsbeutel oder Laparoskopiebeutel bezeichnet wird, wird zur Entnahme von Gewebe oder auch Körperflüssigkeit aus dem menschlichen oder tierischen Körper verwendet.

Insbesondere bei endoskopischen Eingriffen im menschlichen Körper, die durch eine kleine künstlich geschaffene oder natürliche Öffnung hindurch erfolgen, findet ein solcher endochirurgischer Bergebeutel Anwendung. Beispielsweise bei der Entfernung von Tumorgewebe aus dem Bauchraum ist es wichtig, dass bei der Entnahme des Tumorgewebes dies nicht mit gesundem bzw. unbeteiligtem Gewebe in Berührung kommt, um eine Metastasierung und damit eine Gefährdung des Patienten zu vermeiden. Wenn Tumorgewebe oder Gewebeteile entfernt werden müssen, werden sie daher am Ort, wo sie vom umliegenden Gewebe abgetrennt wurden, in einen Bergebeutel eingebracht, und anschließend wird der Bergebeutel, üblicherweise durch einen Trokar hindurch, aus dem Körper herausgezogen. Der Bergebeutel wird entsprechend vor dem Einbringen des Gewebes in den Bergebeutel, üblicherweise durch einen Trokar, in den Körper an Ort und Stelle gebracht.

Eine wichtige Anforderung an einen solchen Bergebeutel besteht darin, dass das im Bergebeutel aufgenommene Gewebe vor dem Herausziehen des Bergebeutels aus dem Körper nicht entweichen kann. Aus diesem Grund wurden Bergebeutel geschaffen, insbesondere ein solcher, wie er in dem oben genannten Dokument EP 0 578 997 B1 beschrieben ist, die an ihrer Einfüllöffnung, durch die das Gewebe in den Bergebeutel eingebracht wird, Zugschnüre zum Verschließen der Öffnung aufweisen. Trotz einer solchen Verschlussmöglichkeit ist eine sichere Dichtigkeit des Bergebeutels nicht in jedem Fall gewährleistet.

Ein weiterer Bergebeutel ist aus dem Dokument EP 0 696 899 B1 bekannt. Dieser Bergebeutel besteht aus einem Beutel mit zwei Öffnungen, und zwar einer kleinen Öffnung und einer größeren Öffnung. Die Öffnungen befinden sich jeweils, in Längsrichtung des Bergebeutels gesehen, an einem Ende des Bergebeutels. Durch das Vorsehen zweier gegenüberliegender Öffnungen soll es ermöglicht werden, dass durch die kleinere Öffnung ein Greifinstrument hindurchgeführt wird, um in dem Beutel durch die große Öffnung eingebrachtes Gewebe zerkleinern zu können. Für beide Öffnungen sind wiederum Zugschnüre zum Verschließen der Öffnungen vorgesehen, was, wie bei dem zuvor beschriebenen bekannten Bergebeutel, den Nachteil einer nicht ausreichenden Dichtigkeit mit sich bringt.

Ferner ist aus dem Dokument US 6,206,889 B1 ein Bergebeutel mit nur einer Öffnung bekannt, die als Einfüllöffnung zum Einfüllen von Gewebestücken dient. Der Rand der Öffnung ist mit einem flexiblen Element versehen, das die Öffnung nach Einführen des Bergebeutels mittels eines Applikators selbsttätig aufspreizt.

Ein weiterer Nachteil der bekannten Bergebeutel besteht darin, dass sie insbesondere für ihre Verwendung in der Pathologie unnötig groß sind, da für Pathologiezwecke nur kleine Gewebestückchen entnommen werden, um diese nach der Entnahme aus dem Körper zu untersuchen.

Der Erfindung liegt die Aufgabe zugrunde, einen Bergebeutel der eingangs genannten Art bereitzustellen, in dem Körpergewebe oder -flüssigkeit möglichst entweichungssicher eingebracht werden kann, so dass der Inhalt beim Entfernen des Bergebeutels sicher aus dem Körper entnommen werden kann.

Erfindungsgemäß wird die Aufgabe hinsichtlich des eingangs genannten endochirurgischen Bergebeutels dadurch gelöst, dass in dem Beutelhauptteil eine Innenhülle angeordnet ist, die mit dem Beutelhauptteil verbunden ist und sich zur ersten Einfüllöffnung hin öffnet, an den Beutelhauptteil jedoch bezüglich der ersten Einfüllöffnung dichtend anschließt, und die an ihrem von der ersten Einfüllöffnung abgewandten zweiten Ende, das von einem von der ersten Einfüllöffnung abgewandten Ende des Beutelhauptteils beabstandet ist, eine zweite Einfüllöffnung aufweist, durch die das Gewebe bzw. die Flüssigkeit in den Beutelhauptteil eingebracht werden kann.

Der erfindungsgemäße Bergebeutel weist demnach einen Beutelhauptteil sowie eine darin angeordnete Innenhülle auf. Die Innenhülle weist zwei offene Enden auf, wobei das eine offene Ende die erste Einfüllöffnung bildet, so dass Gewebe oder Flüssigkeit durch diese Öffnung hindurch zunächst in die Innenhülle geführt werden kann, wobei dieses Gewebe dann durch die zweite Einfüllöffnung am zweiten Ende der Innenhülle hindurch in den Beutelhauptteil eingebracht werden kann. Die in dem Beutelhauptteil angeordnete Innenhülle wirkt mit ihrem zweiten Ende als eine Art Ventil, dessen Funktionsprinzip demjenigen einer Reuse entspricht. Gewebe oder Flüssigkeit kann entsprechend durch die Innenhülle und durch deren zweites Ende hindurch in den Beutelhauptteil bzw. den Teil des Beutelhauptteils eingebracht werden, der nicht durch die Innenhülle eingenommen wird und somit ein Reservoir für das Gewebe oder die Flüssigkeit darstellt. Das im Beutelhauptteil bzw. Reservoir befindliche Gewebe kann jedoch umgekehrt ohne Fremdeingriff nicht von selbst wieder durch die zweite Einfüllöffnung in den Innenraum der Innenhülle eintreten und damit aus dem Bergebeutel insgesamt austreten. Auf diese Weise wird bereits ohne zusätzliche Verschlussmittel für die erste Einfüllöffnung, wie Zugschnüre oder dergleichen, eine sehr hohe Dichtigkeit des Bergebeutels erreicht. Da auf Zugschnüre oder dergleichen zum Verschließen der Einfüllöffnung des Beutelhauptteils prinzipiell verzichtet werden kann, ist auch die Handhabung des erfindungsgemäßen Bergebeutels gegenüber den bekannten Bergebeuteln wesentlich vereinfacht, da der bei den bekannten Bergebeuteln zusätzliche Schritt des Verschließens der ersten oder zweiten Einfüllöffnung des Bergebeutels entfallen kann, zumal dieser Schritt bei den bekannten Bergebeuteln im Körper und damit schwer zugänglich erfolgen muss.

In einer bevorzugten Ausgestaltung dient der Raum zwischen der Außenseite der Innenhülle und der Innenseite der Hülle des Beutelhauptteils als Reservoir für das Körpergewebe bzw. die Körperflüssigkeit.

Ein Vorteil dieser Maßnahme besteht darin, dass das Körpergewebe bzw. die Körperflüssigkeit in den Raum zwischen der Außenseite der Innenhülle und der Innenseite der Hülle des Beutelhauptteils eingebracht werden kann und somit ein Entweichen des Gewebes bzw. der Flüssigkeit noch sicherer vermieden werden kann, weil das Gewebe in diesem Reservoir gefangen ist.

In einer weiteren bevorzugten Ausgestaltung ist die Innenhülle trichterförmig ausgebildet.

Diese Maßnahme hat den Vorteil, dass das Einführen von Gewebe durch die Innenhülle in den Beutelhauptteil hinein vereinfacht ist, weil die Instrumentenspitze des Instruments, mit dem das Gewebe gefasst wird, durch die trichterförmige Ausgestaltung der Innenhülle eine Art Zwangsführung in den Beutelhauptteil bzw. das Reservoir hinein erfährt.

In diesem Zusammenhang ist es bevorzugt, wenn das zweite (innere) Ende der Innenhülle quer zur Längsrichtung schmaler als der Beutelhauptteil auf Höhe dieses zweiten Endes ist.

Durch diese Maßnahme wird die Ventilfunktion der Innenhülle noch weiter verbessert, insbesondere wird die Gefahr eines Wiedereintretens eines bereits in den Beutelhauptteil bzw. das Reservoir eingebrachten Gewebestücks in die Innenhülle und damit ein Austreten des Gewebes aus dem Bergebeutel insgesamt verringert, weil der Querschnitt der zweiten Einfüllöffnung gegenüber dem Querschnitt des Beutelhauptteils kleiner ist.

In einer weiteren bevorzugten Ausgestaltung befindet sich das zweite Ende der Innenhülle in einem Abstand von der ersten Einfüllöffnung, der im Bereich von etwa ein Viertel bis etwa drei Viertel des Abstands der ersten Einfüllöffnung vom der ersten Einfüllöffnung gegenüberliegenden Ende des Beutelhauptteils liegt.

Die Innenhülle erstreckt sich aufgrund dieser Ausgestaltung somit im Wesentlichen von der ersten Einfüllöffnung bis in einen mittleren Bereich des Innenraums des Beutelhauptteils, ohne jedoch bis zum gegenüberliegenden Ende des Beutelhauptteils zu reichen, so dass einerseits sichergestellt ist, dass das einzubringende Gewebe die zweite Einfüllöffnung der Innenhülle vollständig passiert und nicht noch teilweise in der Innenhülle liegt, wenn das Instrument bereits wieder aus dem Bergebeutel zurückgezogen wurde. Andererseits ist sichergestellt, dass das Gewebe möglichst tief in das Innere des Bergebeutels eingeführt werden muss, wodurch die Gefahr einer Fehlhandhabung des Bergebeutels ausgeschlossen oder zumindest minimiert ist. Außerdem wird die Reusenfunktion der Innenhülle durch diese Maßnahme weiter verbessert.

In einer weiteren bevorzugten Ausgestaltung ist die Innenhülle nur in ihrem der ersten Einfüllöffnung zugewandten Bereich mit der Hülle des Beutelhauptteils verbunden.

Gemäß dieser Ausgestaltung ist demnach die Innenhülle bezüglich der Hülle des Beutelhauptteils im Wesentlichen lose, so dass eine Gewebeprobe insbesondere zwischen die Außenseite der Innenhülle und die Innenseite der Hülle des Beutelhauptteils gelangen kann, wodurch ein Entweichen der Gewebeprobe aus dem Beutelhauptteil noch sicherer vermieden werden kann. Es versteht sich dabei, dass der der ersten Einfüllöffnung zugewandte Bereich der Innenhülle mit der Hülle des Beutelhauptteils vollumfänglich und dicht, insbesondere flüssigkeitsdicht, verbunden ist.

In einer weiteren bevorzugten Ausgestaltung ist die Innenhülle mit der Hülle des Beutelhauptteils einstückig verbunden.

Diese Maßnahme ist vorteilhaft bei der Herstellung des Bergebeutels, da die Innenhülle nicht mit der Hülle des Beutelhauptteils durch eine Verbindungstechnik, wie Schweißen oder Kleben, verbunden werden muss.

In einer weiteren bevorzugten Ausgestaltung ist die Innenhülle durch eine Einstülpung eines Abschnitts der Hülle des Beutelhauptteils gebildet.

Diese Maßnahme hat den besonderen Vorteil, dass die Herstellung des erfindungsgemäßen Bergebeutels mit Beutelhauptteil und Innenhülle sehr einfach und somit besonders kostengünstig ist.

In einer weiteren bevorzugten Ausgestaltung ist die erste Einfüllöffnung verschließbar.

Wie bereits oben erwähnt, sind Verschlussmittel zum Verschließen der ersten Einfüllöffnung aufgrund der erfindungsgemäßen Ausgestaltung des Bergebeutels wegen der Reusenfunktion der Innenhülle normalerweise nicht erforderlich, können aber beispielsweise für spezielle Anwendungen, insbesondere für Flüssigkeiten vorgesehen sein.

Die Verschließbarkeit der Einfüllöffnung dient auch weniger dem Verhindern eines Entweichens von Gewebe während des Herausziehens des Bergebeutels aus dem Körper, sondern kann beispielsweise vorteilhaft sein, wenn der Bergebeutel mit dem darin enthaltenen Gewebe noch eine gewisse Zeit gelagert werden soll, wodurch ein Eindringen von Kontaminationen in das Innere der Innenhülle und damit in den Beutelhauptteil vermieden werden kann, ebenso wird eine Kontamination des (gesunden) Körpergewebes durch Gewebestücke, die sich im Beutelhauptteil (Reservoir) des Bergebeutels befinden, vermieden.

Zum erleichterten Manipulieren des Bergebeutels im Inneren des Körpers sind vorzugsweise Mittel zum Greifen des Bergebeutels mit einem Instrument, beispielsweise einer Greifzange, vorgesehen, wobei die Mittel zum Greifen vorzugsweise einen Faden, eine Fadenschlaufe oder ein Auge in der Hülle aufweisen, wobei das zuvor genannte Auge dann allerdings an einer Stelle der Hülle des Beutelhauptteils angeordnet ist, die die Dichtigkeit des Beutelhauptteils nicht beeinträchtigt.

Hierzu ist gegebenenfalls eine zusätzliche dichtende Verbindung der Innenhülle und des Beutelhauptteils auf der Seite des Auges vorzusehen, die der ersten Einfüllöffnung des Bergebeutels gegenüberliegt.

In einer weiteren bevorzugten Ausgestaltung ist die Hülle des Beutelhauptteils und/oder die Innenhülle aus zwei im Wesentlichen flach aufeinanderliegenden Folien gebildet.

Hierbei ist von Vorteil, dass der Bergebeutel insgesamt eine sehr geringe Dicke hat und sich insbesondere zu einer Rolle mit geringem Durchmesser aufrollen lässt, wodurch das Einbringen des Bergebeutels in den Körper vereinfacht wird. Des Weiteren hat diese Maßnahme hinsichtlich der Innenhülle den weiteren vorteil, dass die zweite Einfüllöffnung als sehr schmaler Spalt ausgebildet ist, der sich beim Einbringen von Gewebe mittels eines Instruments leicht aufweiten lässt, nach dem Herausziehen des Instruments aus der Innenhülle sich jedoch selbst wieder zu einem sehr schmalen Spalt zurückbildet, wodurch die reusenartige Ventilfunktion der Innenhülle noch weiter verbessert wird.
In einer weiteren bevorzugten Ausgestaltung ist der Bergebeutel so ausgebildet, dass er aufrollbar ist und vom aufgerollten Zustand im Wesentlichen selbständig in einen flach ausgebreiteten entrollten Zustand übergeht.

Hierbei ist von Vorteil, dass die Handhabung des Bergebeutels beim Einbringen in den Körper vereinfacht ist, weil sich der Bergebeutel beispielsweise durch einen Trokar oder einen hiernach noch zu beschreibenden erfindungsgemäßen Applikator im aufgerollten Zustand leicht einbringen lässt und nach dem Verlassen des Trokars bzw. des Applikators im Körper selbständig entrollt, ohne dass dazu umständliche Manipulationen erforderlich sind.

Weitere Vorteile und Merkmale ergeben sich aus der nachfolgenden Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden mit Bezug auf diese hiernach näher beschrieben. Es zeigen:
- Fig. 1: einen endochirurgischen Bergebeutel in Seitenansicht;
- Fig. 2: einen Schnitt durch den Bergebeutel entlang der Linie II-II in Fig. 1;
- Fig. 3: einen Schnitt entlang der Linie III-III durch den Bergebeutel in Fig. 1;
- Fig. 4: den Bergebeutel in Fig. 1 in einem vorhergehenden Herstellungsstadium;
- Fig. 5: ein gegenüber dem Bergebeutel in Fig. 1 geringfügig abgewandeltes Ausführungsbeispiel eines Bergebeutels;
- Fig. 6: eine schematische Darstellung des Einbringens von Gewebe in den Bergebeutel in Fig. 1;
- Fig. 7a) und b): einen Applikator zum Einbringen eines Bergebeutels in den Körper, wobei Fig. 7a) ein Rohr und Fig. 7b) einen Stößel des Applikators zeigen; und
- Fig. 8: den Applikator in Fig. 7 bei seiner Verwendung zum Einbringen eines Bergebeutels in den Körper.

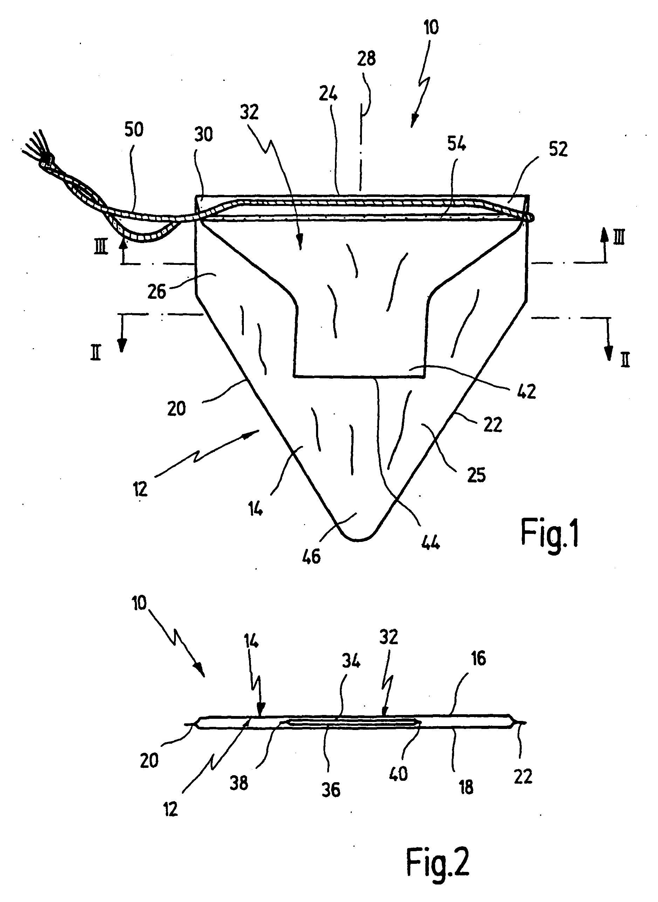

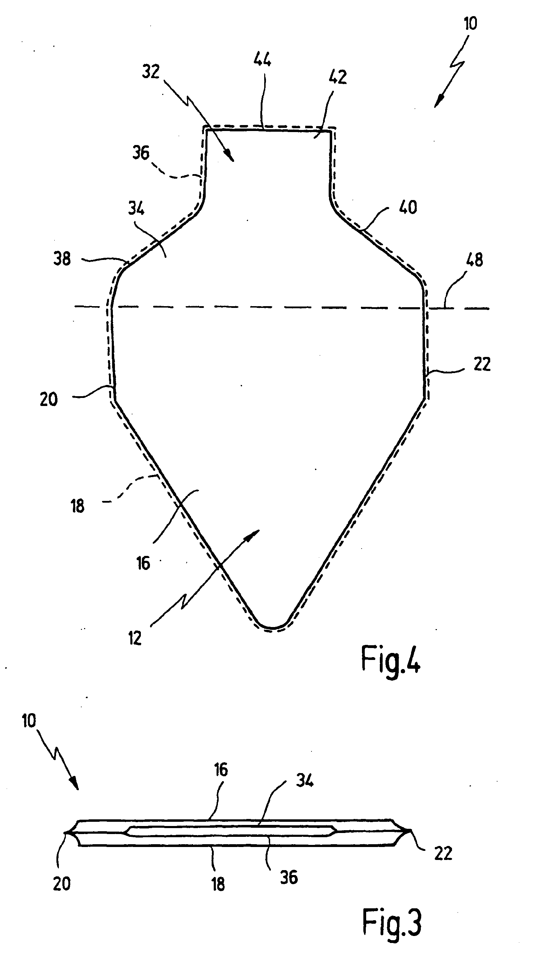

In Figuren 1 bis 3 ist ein mit dem allgemeinen Bezugszeichen 10 versehener endochirurgischer Bergebeutel für die Aufnahme von Körpergewebe oder -flüssigkeit dargestellt. Der Bergebeutel 10 wird vorrangig für pathologische Zwecke verwendet. Mit dem Bergebeutel 10 kann darin intrakorporal eingebrachtes Gewebe aus dem Körper entnommen und anschließend pathologisch untersucht werden.

Der Bergebeutel 10 weist einen Beutelhauptteil 12 auf, der aus einer flexiblen Hülle 14 gebildet ist. Die Hülle 14 ist transparent und besteht beispielsweise aus Polymethylen bzw. Polymethan.

Die Hülle 14 ist aus zwei flach aufeinanderliegenden Folien 16 und 18 gebildet, die an ihren seitlichen Rändern 20 und 22 fest miteinander verbunden sind, vorzugsweise durch Verschweißen bzw. Heißsiegeln.

An einen im Wesentlichen dreieckigen Abschnitt 25 des Beutelhauptteils 12 schließt sich ein im Wesentlichen rechteckiger Abschnitt 26 des Beutelhauptteils 12 an.

Der Bergebeutel 10 weist ferner eine erste Einfüllöffnung 24 auf, durch die Gewebe oder Flüssigkeit in den Beutelhauptteil 12 eingebracht werden kann, wie später noch beschrieben wird, ohne dass der Innenraum des Beutelhauptteils bzw. eines Reservoirs 12, das später noch beschrieben wird, an der ersten Einfüllöffnung 24 zur Außenseite des Bergebeutels 10 hin offen ist, vielmehr ist der Beutelhauptteil 12 auch an der Einfüllöffnung 24 dicht.

Die erste Einfüllöffnung 24 erstreckt sich quer zu einer Längsachse oder Längsrichtung 28 des Bergebeutels 10 über die gesamte Breite des Bergebeutels 10. Die Einfüllöffnung 24 befindet sich an einem Ende 30 des Bergebeutels 10.

Der Beutelhauptteil bzw. das Reservoir 12 ist entlang seiner Ränder 20, 22 und entlang der ersten Einfüllöffnung 24 dicht, insbesondere flüssigkeitsdicht geschlossen.

In dem Beutelhauptteil 12 ist eine Innenhülle 32 angeordnet, die sich von der Einfüllöffnung 24 ausgehend in der Längsrichtung 28 des Beutelhauptteils 12 in das Innere des Beutelhauptteils 12 erstreckt. Die Innenhülle 32 öffnet sich zur Einfüllöffnung 24 hin, so dass durch die Einfüllöffnung 24 eingebrachtes Gewebe zunächst in das Innere der Innenhülle 32 gelangt.

Die Innenhülle 32 ist wie die Hülle 14 aus zwei flach aufeinanderliegenden Folien 34 und 36 gebildet, die an ihren Rändern 38 und 40 fest miteinander verbunden sind, ebenfalls beispielsweise durch Verschweißen bzw. Heißsiegeln. Die Folien 34, 36 sind transparent und bestehen aus dem gleichen Material wie der Beutelhauptteil 12, im vorliegenden Fall aus Polyurethan. Alternativ kann die Innenhülle 32 auch aus einem anderen Material als der Beutelhauptteil 12 bestehen, so dass die Paarung aus Innenhülle 32 und Beutelhauptteil 12 durch aufeinander abgestimmte Materialien sowie deren Festigkeit und Dicke ein besonders gutes Abdichten bzw. einen guten Reuseneffekt, wie später noch beschrieben wird, gewährleistet.

Während die Innenhülle 32 den Beutelhauptteil 12 an der Einfüllöffnung 24 dicht abschließt, weist die Innenhülle 32 an einem der Einfüllöffnung 24 abgewandten Ende 42 eine zweite Einfüllöffnung 44 auf, so dass durch die Einfüllöffnung 24 in das Innere der Innenhülle 32 eingeführtes Gewebe durch die Einfüllöffnung 44 in den Beutelhauptteil 12 eingebracht werden kann.

Das Ende 42 der Innenhülle 32 ist von einem der Einfüllöffnung 24 gegenüberliegenden Ende 46 des Beutelhauptteils 12 ausreichend beabstandet.

Allgemein befindet sich das Ende 42 der Innenhülle 32 in einem Abstand von der Einfüllöffnung 24, der im Bereich von etwa ein Viertel bis etwa drei Viertel des Abstands der Einfüllöffnung 24 vom der Einfüllöffnung 24 entgegengesetzten Ende 46 des Beutelhauptteils 12 liegt. Im gezeigten Ausführungsbeispiel befindet sich das Ende 42 der Innenhülle 32 etwa hälftig in Bezug auf die Längsrichtung 28 zwischen der Einfüllöffnung 24 und dem Ende 46 des Beutelhauptteils 12.

Die Innenhülle 32 verjüngt sich von der Einfüllöffnung 24 zur Einfüllöffnung 44 trichterförmig, wobei das Ende 42 der Innenhülle 32 quer zur Längsrichtung 28 schmaler ist als der Beutelhauptteil 12 auf Höhe des Endes 42 der Innenhülle 32. An der Einfüllöffnung 24 erstreckt sich die Innenhülle 32 quer zur Längsrichtung 28 über die gesamte Breite des Bergebeutels 10.

Die Innenhülle 32 ist nur im Bereich der Einfüllöffnung 24 mit der Hülle 14 des Beutelhauptteils 12 verbunden, so dass die Innenhülle 32 bezüglich der Hülle 14 des Beutelhauptteils 12 außer im Bereich der Einfüllöffnung 24 lose ist. Auf diese Weise ist auch der Raum zwischen der Außenseite der Innenhülle 32 und der Innenseite der Hülle 14 im Bereich der Innenhülle 32 für die Aufnahme von Gewebe oder Flüssigkeit nutzbarer Innenraum des Beutelhauptteils 12, das Reservoir, das, wie bereits erwähnt, zur Einfüllöffnung 24 hin dicht abgeschlossen ist, und zwar aufgrund der Innenhülle 32.

Die Innenhülle 32 ist insbesondere mit der Hülle 14 des Beutelhauptteils 12 einstückig verbunden, d.h. die Folien 16, 18 des Beutelhauptteils 12 und die Folien 34, 36 der Innenhülle 32 sind einstückig bzw. einteilig miteinander ausgebildet.

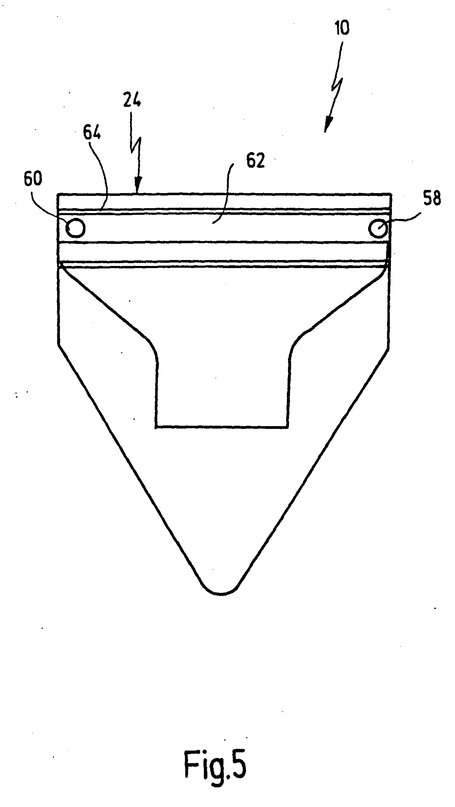

In Fig. 4 ist der Bergebeutel 10 in einem Herstellungsvorstadium dargestellt.

Der Bergebeutel wird dadurch hergestellt, dass zwei gleiche Zuschnitte von Folien flach aufeinandergelegt werden, die mit Ausnahme des Bereichs der Einfüllöffnung 44 an den Rändern 20, 22 bzw. 38, 40 fest und dicht miteinander verbunden werden, beispielsweise verschweißt bzw. heißgesiegelt oder geklebt werden. Alternativ könnte die Ausgangsform für den Bergebeutel auch ursprünglich bereits schlauchartig mit einem geschlossenen Ende sein.

Die Zuschnitte bzw. die schlauchartige Ausgangsform weisen bereits die spätere Form des Beutelhauptteils 12 und der Innenhülle 32 auf, wie in Fig. 4 dargestellt ist.

Auf diese Weise sind der Beutelhauptteil 12 und die Innenhülle 32 bereits von vornherein einteilig ausgebildet.

Nach dem festen Verbinden der Ränder 20, 22, 38, 40 wird dann die Innenhülle 32 entlang etwa einer Linie 48 nach innen zwischen die Folien 16 und 18 des Beutelhauptteils 12 eingestülpt, wodurch der Bergebeutel 10 mit der Innenhülle 32 gemäß Fig. 1 entsteht.

Vor dem Einstülpen der Innenhülle 32 in den Beutelhauptteil 12 wird noch eine Fadenschlaufe 50 zwischen die Folien 16, 18 bzw. 34, 36 gelegt, vorzugsweise bevor die Ränder 20, 22 bzw. 38, 40 fest miteinander verbunden werden, so dass die beiden freien, noch zu verbindenden Enden der Fadenschlaufe bzw. ein Abschnitt der bereits geschlossenen Fadenschlaufe nach dem Verbinden der Ränder 20, 22 bzw. 38, 40 nach außen herausragen. Bei der alternativen Ausgangsform für den Bergebeutel in Art eines einseitig geschlossenen Schlauchs müsste ein Durchgang für die Fadenschlaufe durch die Ausgangsform für den Bergebeutel geschaffen werden, an einer Stelle, die etwas auf der Seite des geschlossenen Endes der Ausgangsform für den Bergebeutel liegt, gesehen von der Stelle, an der der Bergebeutel eingestülpt werden soll. Die Fadenschlaufe 50 kommt dann nach dem Einstülpen der Innenhülle 32 um die Fadenschlaufe 50 herum in das Innere des Beutelhauptteils 12 in einer Tasche 52 zu liegen, die durch eine Befestigungsnaht 54, die durch Heißsiegeln oder Kleben gebildet werden kann, begrenzt ist. Die Befestigungsnaht 54 wird nach dem Einstülpen der Innenhülle 32 angebracht und kann außerdem ein Wiederausstülpen der Innenhülle 32 aus dem Beutelhauptteil 12 heraus verhindern.

Die Fadenschlaufe 50 dient dazu, den Bergebeutel 10 mit einem Instrument, beispielsweise einer Fasszange, greifen zu können, und stellt insoweit ein Mittel zum Greifen des Bergebeutels 10 dar, und dient insbesondere nicht als Zugschnur zum Verschließen der Einfüllöffnung 24 wie bei den bekannten Bergebeuteln, weil ein derartiges Verschließen bei dem Bergebeutel 10 prinzipiell nicht erforderlich ist, um ein Entweichen einer in den Beutelhauptteil 12 aufgenommenen Gewebeprobe oder Flüssigkeit zu verhindern.

Alternativ oder zusätzlich zu der Fadenschlaufe 50 kann gemäß Fig. 5 als Mittel zum Greifen des Bergebeutels 10 im Körper ein Auge 58 oder ein weiteres Auge 60 vorgesehen sein, das bzw. die an einem Hüllenfortsatz 62 angebracht ist/sind, so dass die Augen 58 und 60 nicht die Dichtigkeit des Beutelhauptteils 12 beeinträchtigen, speziell kann der Hüllenfortsatz 62, wie zum Ausführungsbeispiel mit der Fadenschlaufe 50 beschrieben, durch eine Befestigungsnaht von dem Auge 58 bzw. den Augen 58 und 60 aus gesehen auf der geschlossenen Seite des Beutelhauptteils 12 abgedichtet sein.

An dem Hüllenfortsatz 62 kann weiterhin noch ein Verschlussmittel 64 zum Verschließen der Einfüllöffnung 24 vorgesehen sein. Ein solches Verschlussmittel 64 kann beispielsweise ein bei Lebensmittelbeuteln bekannter so genannter Zipp-Verschluss sein. Die Verschließbarkeit der Einfüllöffnung 24 dient, wie bereits erwähnt, weniger der Verhinderung eines Entweichens von in dem Beutelhauptteil 12 aufgenommenem Gewebe oder Flüssigkeit extrakorporal, sondern dient beispielsweise der Verhinderung des Eindringens von Kontaminationen in den Beutelhauptteil bzw. das Reservoir 12, beispielsweise wenn der Bergebeutel 10 vor der Entnahme des Gewebes aus dem Bergebeutel 10 eine gewisse Zeit gelagert wird, und auch von Kontaminationen von (gesundem) Körpergewebe durch das zu entnehmende Gewebe oder die zu entnehmende Flüssigkeit.

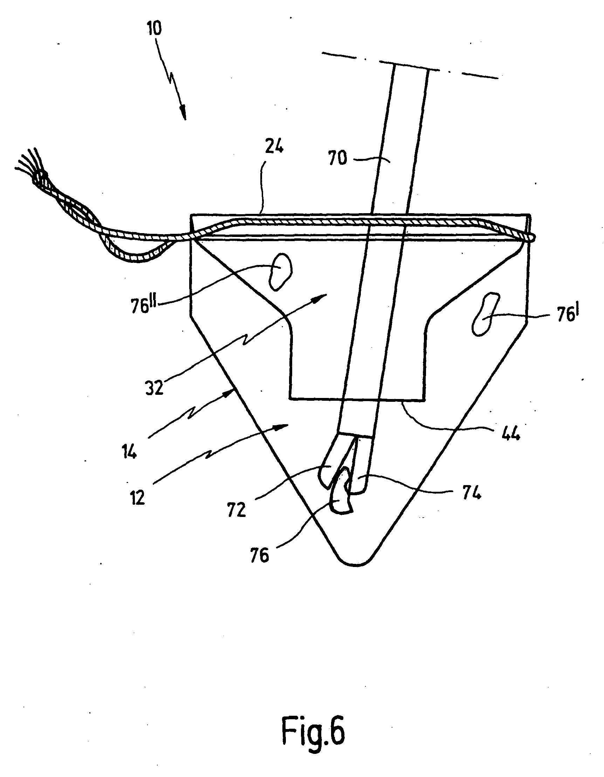

Mit Bezug auf Fig. 6 wird nun das Einbringen von Körpergewebe oder -flüssigkeit in den Bergebeutel 10 beschrieben.

In Fig. 6 ist schematisch ein distaler Bereich eines Instruments 70 dargestellt, das am distalen Ende Maulteile 72 und 74 aufweist, mit denen ein Gewebestück 76 gefasst wird. Zum Einbringen des Gewebestücks 76 in den Beutelhauptteil 12 wird das Instrument 70 mit den Maulteilen 72, 74 voran durch die Einfüllöffnung 24, von dort durch das Innere der Innenhülle 32 und dann durch die Öffnung 44 der Innenhülle 32 in den Beutelhauptteil bzw. das Reservoir 12 eingebracht. Das Gewebestück 76 wird anschließend durch öffnen der Maulteile 72, 74 losgelassen und das Instrument 70 aus dem Bergebeutel 10 herausgezogen.

Die Innenhülle 32 wirkt nun wie eine Art Ventil mit der Funktionsweise einer Reuse, d.h. durch die Öffnung 44 kann Gewebe in den Beutelhauptteil 12 eingebracht werden, von sich aus kann eingebrachtes Gewebe, beispielsweise das Gewebestück 76, nicht wieder aus dem Beutelhauptteil 12 entweichen, weil der Beutelhauptteil 12 allseitig geschlossen ist. Die Öffnung 44 selbst bildet nur einen äußerst schmalen Spalt oder Schlitz, weil die Innenhülle 32 aus zwei flach aufeinanderliegenden Folien gebildet ist, so dass selbst dann, wenn der Bergebeutel 10 mit der Einfüllöffnung 24 nach unten gehalten wird, das Gewebestück 76 die Öffnung 44 nicht wieder passieren kann. Das Gewebestück 76 kann sich dabei zwar an eine mit 76' dargestellte Position oder auch an eine mit 76" dargestellte Position bewegen, wenn der Bergebeutel 10 mit der Einfüllöffnung 24 nach unten gehalten wird, kann jedoch aufgrund dessen, dass der Beutelhauptteil 12 zur Einfüllöffnung 24 hin nicht offen ist, nicht aus dem Beutelhauptteil 12 entweichen. Die mit 76" dargestellte Position des Gewebestücks 76 ist so zu verstehen, dass sich das Gewebestück 76 in diesem Fall zwischen der Außenseite der Innenhülle 32 und der Innenseite der Hülle 14 des Beutelhauptteils 12 befindet.

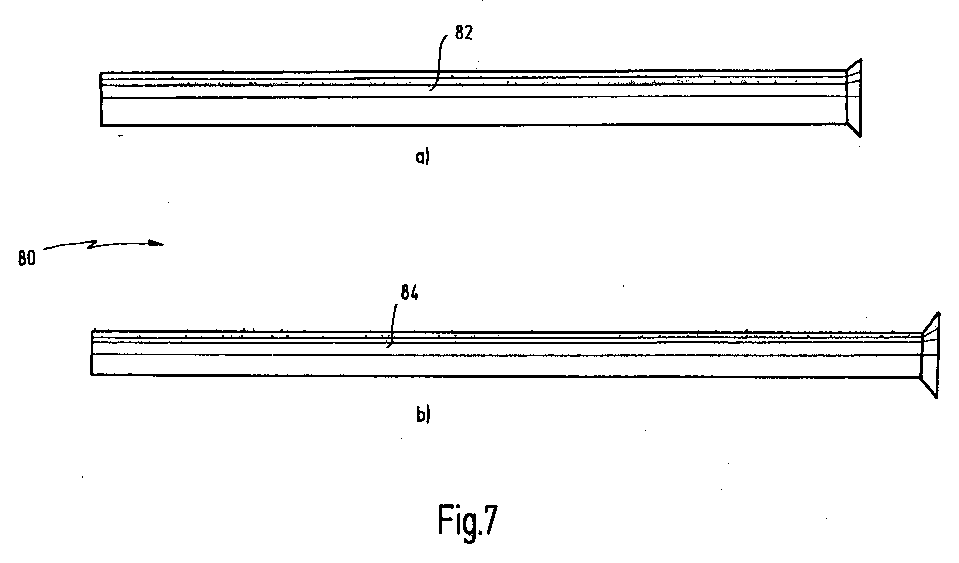

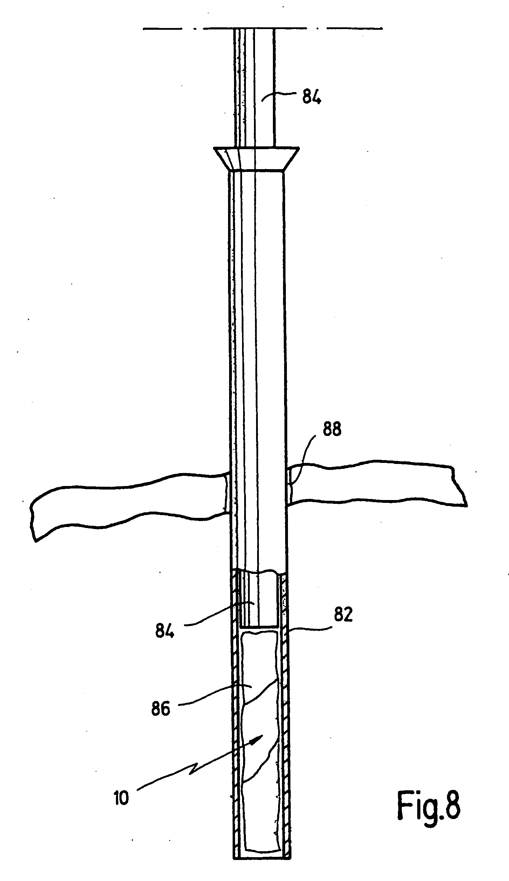

Mit Bezug auf Figuren 7 und 8 wird nachfolgend ein Applikator 80 beschrieben, mit dem der Bergebeutel 10 in den Körper eingebracht werden kann.

Der Applikator 80 weist ein Rohr 82 von zylindrischer Form auf, sowie einen Stößel 84, ebenfalls von zylindrischer Form, der als Rohr oder Stab ausgebildet sein kann, wobei der Außendurchmesser des Stößels 84 an den Innendurchmesser des Rohrs 82 derart angepasst ist, dass der Stößel 84 in das Rohr 82 einsetzbar ist. Der Stößel 84 ist in dem Rohr 82 verschieblich beweglich.

Der Bergebeutel 10 ist parallel zur Längsrichtung 28 oder quer dazu aufrollbar und insbesondere so ausgebildet, dass er sich vom aufgerollten Zustand im Wesentlichen selbständig in einen flach ausgebreiteten entrollten Zustand entfaltet, wenn keine äußeren Kräfte auf den Bergebeutel 10 wirken.

Zum Applizieren bzw. Einbringen des Bergebeutels 10 in den Körper wird der Bergebeutel 10 entsprechend Fig. 8 zu einer Rolle 86 aufgerollt. Die Rolle 86 wird dann in das Rohr 82 eingesetzt, vorzugsweise vom distalen Ende des Rohrs 82 her. Die Länge des Rohrs 82 ist so angepasst, dass in Längsrichtung des Rohrs 82 auch gleichzeitig zwei Rollen 86 von zwei Bergebeuteln 10 hintereinander aufgenommen werden können.

Das Rohr 82 wird anschließend durch eine natürliche oder künstlich geschaffene Körperöffnung 88 in den Körper eingeführt, und zwar an Ort und Stelle, wo der Bergebeutel 10 zur Aufnahme von Gewebe benötigt wird.

Anschließend wird der Stößel 84 von proximal in das Rohr 82 eingesetzt, und durch Verschieben des Rohrs 84 nach distal wird die Rolle 86 aus dem distalen Ende des Rohrs 82 herausgedrückt, und sobald die Rolle 86 das Rohr 82 vollständig verlassen hat, entrollt sich der Bergebeutel 10 aufgrund seiner elastischen Eigenschaften selbständig.

Anschließend kann gemäß Fig. 6 Gewebe wie beschrieben in den Bergebeutel 10 eingebracht werden.

## Patentansprüche

1. Endochirurgischer Bergebeutel für die Aufnahme von Körpergewebe oder -flüssigkeit, mit einem Beutelhauptteil (12), der aus einer flexiblen Hülle (14) gebildet ist, und mit einer ersten Einfüllöffnung (24) zum Einbringen von Gewebe oder Flüssigkeit in den Beutelhauptteil (12), **dadurch gekennzeichnet, dass** in dem Beutelhauptteil (12) eine Innenhülle (32) angeordnet ist, die mit dem Beutelhauptteil (12) verbunden ist und sich zur ersten Einfüllöffnung (24) hin öffnet, an den Beutelhauptteil (12) jedoch bezüglich der ersten Einfüllöffnung (24) dichtend anschließt, und die an ihrem von der ersten Einfüllöffnung (24) abgewandten zweiten Ende (42), das von einem von der ersten Einfüllöffnung (24) abgewandten Ende (46) des Beutelhauptteils (12) beabstandet ist, eine zweite Einfüllöffnung (44) aufweist, durch die das Gewebe bzw. die Flüssigkeit in den Beutelhauptteil (12) eingebracht werden kann.

2. Bergebeutel nach Anspruch 1, **dadurch gekennzeichnet, dass** der Raum zwischen der Außenseite der Innenhülle (32) und der Innenseite der Hülle (14) als Reservoir für das Körpergewebe bzw. die Körperflüssigkeit dient.

3. Bergebeutel nach Anspruch 1 oder 2, **dadurch gekennzeichnet, dass** die Innenhülle (32) trichterförmig ausgebildet ist.

4. Bergebeutel nach einem der Ansprüche 1 bis 3, **dadurch gekennzeichnet, dass** sich das zweite Ende (42) der Innenhülle (32) in einem Abstand von der ersten Einfüllöffnung (24) befindet, der im Bereich von etwa ein Viertel bis etwa drei Viertel des Abstands der ersten Einfüllöffnung (24) vom der ersten Einfüllöffnung (24) gegenüberliegenden Ende (46) des Beutelhauptteils (12) liegt.

5. Bergebeutel nach einem der Ansprüche 1 bis 4, **dadurch gekennzeichnet, dass** die Innenhülle (32) nur in ihrem der ersten Einfüllöffnung (24) zugewandten Bereich mit der Hülle (14) des Beutelhauptteils (12) verbunden ist.

6. Bergebeutel nach einem der Ansprüche 1 bis 5, **dadurch gekennzeichnet, dass** die Innenhülle (32) mit der Hülle (14) des Beutelhauptteils (12) einstückig verbunden ist.

7. Bergebeutel nach einem der Ansprüche 1 bis 6, **dadurch gekennzeichnet, dass** die Innenhülle (32) durch eine Einstülpung eines Abschnitts der Hülle (14) des Beutelhauptteils (12) gebildet ist.

8. Bergebeutel nach einem der Ansprüche 1 bis 7, **dadurch gekennzeichnet, dass** das zweite Ende (42) der Innenhülle (32) quer zur Längsrichtung schmaler als der Beutelhauptteil (12) auf Höhe dieses zweiten Endes (42) ist.

9. Bergebeutel nach einem der Ansprüche 1 bis 8, **dadurch gekennzeichnet, dass** die erste Einfüllöffnung (24) verschließbar ist.

10. Bergebeutel nach einem der Ansprüche 1 bis 9, **gekennzeichnet durch** Mittel zum Greifen des Bergebeutels (10) mit einem Instrument (70).

11. Bergebeutel nach Anspruch 10, **dadurch gekennzeichnet, dass** die Mittel zum Greifen einen Faden, eine Fadenschlaufe (50) oder ein Auge (58) in der Hülle (14) aufweisen.

12. Bergebeutel nach einem der Ansprüche 1 bis 11, **dadurch gekennzeichnet, dass** die Hülle (14) des Beutelhauptteils (12) und/oder die Innenhülle (32) aus zwei im Wesentlichen flach aufeinanderliegenden Folien (16, 18; 34, 36) gebildet ist.

13. Bergebeutel nach einem der Ansprüche 1 bis 12, **dadurch gekennzeichnet, dass** der Bergebeutel (10) so ausgebildet ist, dass er aufrollbar ist und vom aufgerollten Zustand im Wesentlichen selbständig in einen flach ausgebreiteten entrollten Zustand übergeht.

## Claims

1. An endosurgical extraction bag for collecting body tissue or body fluid, comprising a bag main part (12) formed from a flexible envelope (14), and a first admission opening (24) for introducing tissue or fluid into the bag main part (12), **characterized in that**, arranged in the bag main part (12), there is an inner envelope (32) which is connected to the bag main part (12) and opens toward the first admission opening (24), but adjoins the bag main part (12) sealingly in respect of the first admission opening (24), and which has a second admission opening (44) at its second end (42) remote from the first admission opening (24) and spaced apart from an end (46) of the bag main part (12) remote from the first admission opening (24), through which second admission opening (44) the tissue or fluid can be introduced into the bag main part (12).

2. The extraction bag of claim 1, **characterized in that** the space between the outer face of the inner envelope (32) and the inner face of the envelope (14) serves as a reservoir for the body tissue or body fluid.

3. The extraction bag of claim 1 or 2, **characterized in that** the inner envelope (32) has a funnel-shaped configuration.

4. The extraction bag of any one of claims 1 through 3, **characterized in that** the second end (42) of the inner envelope (32) is spaced apart from the first admission opening (24) by a distance which is in the region of approximately one quarter to approximately three quarters of the distance of the first admission opening (24) from that end (46) of the bag main part (12) lying remote from the first admission opening (24).

5. The extraction bag of any one of claims 1 through 4, **characterized in that** the inner envelope (32) is connected to the envelope (14) of the bag main part (12) only in its area directed toward the first admission opening (24).

6. The extraction bag of any one of claims 1 through 5, **characterized in that** the inner envelope (32) is connected integrally to the envelope (14) of the bag main part (12).

7. The extraction bag of any one of claims 1 through 6, **characterized in that** the inner envelope (32) is formed by turning inward of a section of the envelope (14) of the bag main part (12).

8. The extraction bag of any one of claims 1 through 7, **characterized in that** the second end (42) of the inner envelope (32), transverse to the longitudinal direction, is narrower than the bag main part (12) at this second end (42).

9. The extraction bag of any one of claims 1 through 8, **characterized in that** the first admission opening (24) can be closed.

10. The extraction bag of any one of claims 1 through 9, **characterized by** means for gripping the extraction bag (10) by an instrument (70).

11. The extraction bag of claim 10, **characterized in that** the means for gripping comprise a thread, a thread loop (50), or an eyelet (58) in the envelope (14).

12. The extraction bag of any one of claims 1 through 11, **characterized in that** the envelope (14) of the bag main part (12) and/or the inner envelope (32) is formed from two foils (16, 18; 34, 36) lying substantially flat on one another.

13. The extraction bag of any one of claims 1 through 12, **characterized in that** the extraction bag (10) is designed such that it can be rolled up and, from the rolled-up state, converts more or less automatically into an unrolled flat state.

## Revendications

1. Sac de récupération pour endochirurgie destiné à recevoir du tissu corporel ou un liquide corporel, comportant une partie principale (12), qui est formée par une enveloppe (14) flexible, et comportant une première ouverture de remplissage (24) pour l'introduction de tissu ou liquide dans la partie principale (12) du sac, **caractérisé en ce qu'**une pochette intérieure (32) est agencée dans la partie principale (12) du sac, laquelle est assemblée à la partie principale (12) du sac et s'ouvre vers la première ouverture de remplissage (24), est attachée à la partie principale (12) du sac de manière étanche cependant par rapport à la première ouverture de remplissage (24), et laquelle, au niveau de sa deuxième extrémité (42), opposée à la première ouverture de remplissage (24) et située à distance d'une extrémité (46) de la partie principale (12) du sac, opposée à la première ouverture de remplissage (24), comporte une deuxième ouverture de remplissage (44), par laquelle le tissu et/ou le liquide peuvent être introduits dans la partie principale (12) du sac.

2. Sac de récupération selon la revendication 1, **caractérisé en ce que** l'espace entre le côté extérieur de la pochette intérieure (32) et le côté intérieur de l'enveloppe (14) fait fonction de réservoir pour le tissu corporel et/ou le liquide corporel.

3. Sac de récupération selon la revendication 1 ou 2, **caractérisé en ce que** la pochette intérieure (32) est réalisée en forme d'entonnoir.

4. Sac de récupération selon l'une quelconque des revendications 1 à 3, **caractérisé en ce que** la deuxième extrémité (42) de la pochette intérieure (32) est disposée à une distance de la première ouverture de remplissage (24), qui se situe dans une plage allant d'environ un quart jusqu'aux trois quarts de la distance entre la première ouverture de remplissage (24) et l'extrémité (46) de la partie principale (12) du sac, opposée à la première ouverture de remplissage (24).

5. Sac de récupération selon l'une quelconque des revendications 1 à 4, **caractérisé en ce que** la pochette intérieure (32) est assemblée à l'enveloppe (14) de la partie principale (12) du sac uniquement dans sa partie orientée vers la première ouverture de remplissage (24).

6. Sac de récupération selon l'une quelconque des revendications 1 à 5, **caractérisé en ce que** la pochette intérieure (32) est assemblée d'un seul tenant à l'enveloppe (14) de la partie principale (12) du sac.

7. Sac de récupération selon l'une quelconque des revendications 1 à 6, **caractérisé en ce que** la pochette intérieure (32) est formée par un retournement d'une partie de l'enveloppe (14) de la partie principale (12) du sac.

8. Sac de récupération selon l'une quelconque des revendications 1 à 7, **caractérisé en ce que** la deuxième extrémité (42) de la pochette intérieure (32) est, dans le sens transversal au sens longitudinal, plus étroite que la partie principale (12) du sac à la hauteur de cette deuxième extrémité (42).

9. Sac de récupération selon l'une quelconque des revendications 1 à 8, **caractérisé en ce que** la première ouverture de remplissage (24) peut être fermée.

10. Sac de récupération selon l'une quelconque des revendications 1 à 9, **caractérisé par** des moyens pour saisir le sac de récupération (10) avec un instrument (70).

11. Sac de récupération selon la revendication 10, **caractérisé en ce que** les moyens pour saisir le sac comportent un fil, une boucle de fil (50) ou un oeillet (58) pratiqué dans l'enveloppe (14).

12. Sac de récupération selon l'une quelconque des revendications 1 à 11, **caractérisé en ce que** l'enveloppe (14) de la partie principale (12) du sac et/ou la pochette intérieure (32) sont formées par deux feuilles (16, 18 ; 34, 36) posées sensiblement à plat l'une sur l'autre.

13. Sac de récupération Selon l'une quelconque des revendications 1 à 12, **caractérisé en ce que** le sac de récupération (10) est réalisé de telle sorte qu'il peut être enroulé et passe sensiblement automatiquement de l'état enroulé à l'état déroulé déployé à plat.
